# EUROPÄISCHE PATENTSCHRIFT

(11) **EP 0 538 803 B1**
(45) Veröffentlichungstag und Bekanntmachung des Hinweises auf die Patenterteilung: **05.06.1996**
(21) Anmeldenummer: 92117942.0
(22) Anmeldetag: 20.10.1992
(51) Int. Cl.: C07C 39/20, C07C 69/017, C07D 319/08

(54) **Verfahren zur Herstellung von substituierten Vinylbenzolen**
Process for the preparation of substituted vinyl benzenes
Procédé pour la préparation de benzènes vinyliques substitués

(30) Priorität: 23.10.1991 CH 3101/91
(43) Veröffentlichungstag der Anmeldung: 28.04.1993
(73) Patentinhaber: LONZA AG, CH-3945 Gampel/Wallis (CH)
(72) Erfinder: Warm, Aleksander, Dr., Visperterminen,(Kanton Wallis) (CH); Laffan, David, Dr., Visp,(Kanton Wallis) (CH)
(74) Vertreter: Weinhold, Peter, Dr.

(56) Entgegenhaltungen:
- CHEMISCHE BERICHTE Bd. 92, Nr. 11, 1959, WEINHEIM DE Seiten 2958 - 2961 G. MANECKE ET AL. 'Synthese des 1-Vinyl-2. 4.5-trimethyl-3.6-dihydroxy-benzols'
- CHEMICAL ABSTRACTS, vol. 38, no. 21, 10. November 1944, Columbus, Ohio, US; E. ADLER ET AL. 'Formation of 2,4-dimethyl-1,3-benzodioxans and their fission to o-vinylphenols.' Spalte 5839 ;
- CHEMICAL ABSTRACTS, vol. 78, no. 1, 8. Januar 1973, Columbus, Ohio, US; abstract no. 3864f, N.M. MORLYAN ET AL. 'o-Vinylphenol.' Seite 323 ;Spalte 1 ;
- CHEMICAL ABSTRACTS, vol. 83, no. 19, 10. November 1975, Columbus, Ohio, US; abstract no. 164200e, E.P. FOKIN ET AL. '6-Hydroxy-2,4-dimethyl-1,3-benzodioxan.' Seite 557 ;Spalte 1 ;

## Beschreibung

Die Erfindung betrifft ein neues Verfahren zur Herstellung von substituierten Vinylbenzolen der allgemeinen Formel 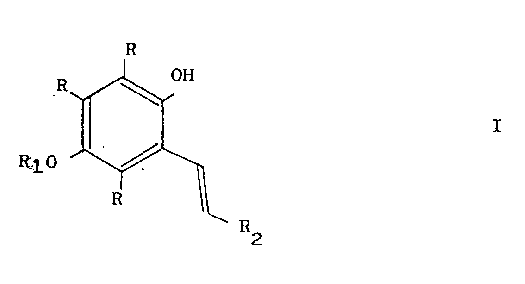 worin Reine Niederalkylgruppe mit 1 bis 4 C-Atomen, R₁ Wasserstoff oder eine Acetylgruppe und R₂ Wasserstoff, eine Niederalkylgruppe mit 1 bis 4 C-Atomen oder eine Benzylgruppe bedeutet.

Substituierte Vinylbenzole sind wertvolle Zwischenprodukte zur Herstellung von Antioxidantien, wie z.B. von Trolox C@ (US-PS 5080 886).

Aus Chem.Berichte 1959, 92, 2958 ist bekannt, 1-Vinyl-2,4,5-trimethyl-3,6-dihydroxybenzol ausgehend, von Trimethylhydrochinon oder von Trimethylphenol,über 4 Stufen herzustellen.

Bei diesem Verfahren erweist sich insbesondere die letzte Stufe, die Decarboxylierung der 3,6-Dihydroxy-2,4,5-trime- thylzimtsäure,als äusserst schwierig.

So konnte das gewünschte Vinylbenzol in einer Ausbeute von lediglich 4%, bezogen auf das eingesetzte Zimtsäurederivat, erhalten werden.

Es bestand daher die Aufgabe, ein Verfahren zu entwickeln, dass diese Nachteile nicht aufweist und mit welchem man in der Lage ist, die subst. Vinylbenzole im technischen Maßstab herzustellen.

Diese Aufgabe konnte gelöst werden mit dem erfindungsgemässen Verfahren nach Patentanspruch 1.

In der ersten Stufe wird erfindungsgemäss ein Trialkylhydrochinon der allgemeinen Formel 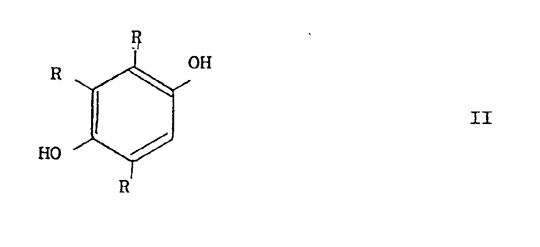 worin R die genannte Bedeutung hat, mit einem Aldehyd der allgemeinen Formel 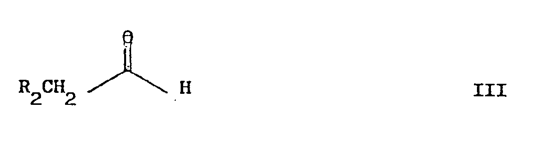 worin R₂ die genannte Bedeutung hat, in Gegenwart einer Säure und unter Ausschluss von Wasser zum Acetal der allgemeinen Formel 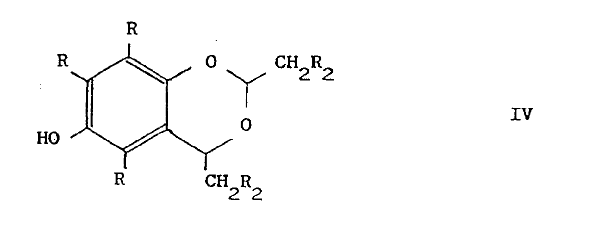 worin R und R₂ die genannte Bedeutung haben, umgesetzt.

Bevorzugt wird Trimethylhydrochinon mit Acetaldehyd zum entsprechenden Acetal der Formel IV,mit R = CH₃ und R₂ = Wasserstoff,umgesetzt.

Die Reaktion verläuft zweckmässig bei einer Temperatur zwischen -30°C,bis 30°C vorzugsweise bei einer Temperatur unter 20°C,in Gegenwart eines inerten Lösungsmittels.

Zweckmässig wird der Aldehyd im Überschuss eingesetzt.

Geeignete wasserfreie Säuren sind z.B. Chlorwasserstoff, Bromwasserstoff, Schwefelsäure oder Methansulfonsäure.

Vorzugsweise wird Chlorwasserstoff verwendet.

Die in guter Ausbeute resultierenden Acetale der allgemeinen Formel IV können auf fachmännische Weise aus dem Reaktionsgemisch isoliert werden.

Die Acetale der allgemeinen Formel sind bislang nicht beschrieben und daher ebenfalls Bestandteil der Erfindung. Bevorzugtes Acetal der allgemeinen Formel IV ist die Verbindung mit R = CH₃ und R₂ = Wasserstoff.

Gewünschtenfalls können die Acetale der allgemeinen Formel IV acetyliert werden zu acetylierten Acetalen der allg. Formel 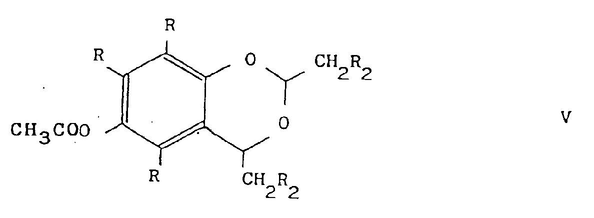 worin R und R₂ die genannte Bedeutung haben. Auch diese Acetale sind bislang nicht beschrieben und daher ebenfalls Bestandteile der Erfindung.

Bevorzugtes acetyliertes Acetal der allg. Formel V ist die Verbindung mit R = CH₃ und R₂ = Wasserstoff.

Die Acetylierung erfolgt zweckmassig mit Acetylchlorid in Gegenwart eines tertiären Amins,wie z. B. Triethylamin,in einem geeigneten inerten Lösungsmittel.

Die Umsetzung verläuft üblicherweise praktisch quantitativ bei einer Temperatur zwischen 0°C und 100°C.

Das acetylierte Acetal kann auf übliche Weise aus dem Reaktionsgemisch isoliert und der Weiterumsetzung zugeführt werden.

In der Folgestufe werden die Acetale der allgemeinen Formel IV oder V zu den subst. Vinylbenzolen der allgemeinen Formel 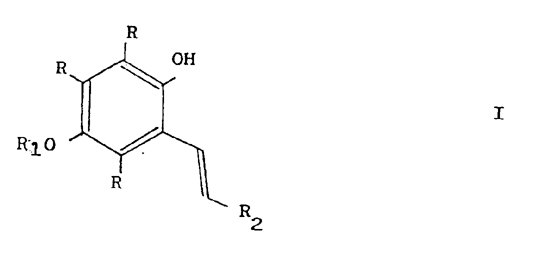 worin R, R₁ und R₂ die genannte Bedeutung haben,pyrolysiert.

Die Pyrolyse erfolgt zweckmässig bei einer Temperatur von über 300°C, vorzugsweise zwischen 400 und 500°C,bei einem reduzierten Druck zwischen 0,5 und 100 mbar.

Den bevorzugten Acetalen der allgemeinen Formel IV bzw. V mit R = CH₃ und R₂ = Wasserstoff entsprechend,resultierten als bevorzugte Vinylbenzole der allg. Formel die methylierten Derivate mit R = CH₃, mit R₁ = Wasserstoff bzw. Acetyl und R₂ = Wasserstoff.

Die acetylierten Vinylbenzole der allgemeinen Formel I mit R₁ = Acetyl sind bislang nicht beschrieben und daher ebenfalls Bestandteil der Erfindung.

Die entsprechenden Vinylbenzole können auf diesem Weg in guter Ausbeute von ca. 70%, bezogen auf die eingesetzten Trialkylhydrochinone, erhalten werden.

### Beispiel 1

### a) Verfahren zur Herstellung von 2.4.5.7.8-Pentamethyl-4H-benzol[1.3]dioxin-6-ol

Trimethylhydrochinon (60,8 g, 0,4 mol) wurde in CH₂Cl₂ (1 I) suspendiert. Zu dieser Suspension wurde eine Lösung von Acetaldehyd (135 ml, 105,6 g, 2,4 mol) in CH₂Cl₂ (280 ml) zugegeben, so dass die Temperatur 20°C nicht überschritt. Dann wurde die Suspension mit gasförmigem HCI gesättigt, bis die Reaktion beendet war (verfolgt mit DC, Toluol/Aceton, 4:1). Während der HCI-Zugabe löste sich die Suspension allmählich. Die gelbe Lösung wurde unter Vakuum eingeengt. Man erhielt 83 g (93,7%) gelben Feststoff mit einem Schmelzpunkt von 101°C- 103°C.
¹H-NMR: (C₆D₆, 300 MHz) δ in ppm 5,21 (q, 1 H, J=7Hz);
   4,87 (q, 1 H, J=6Hz);
   3,78 (s, 1H);
   2,21 (s, 3H);
   1,94 (s, 3H);
   1,69 (s, 3H);
   1,52 (d, 3H, J=5Hz);
   1,28 (d, 3H, J=6Hz)
Isomer: 5,01 (q, 1H, J=6Hz);
   4,83 (g, 1 H, J=5Hz);
   3,82 (s, 3H);
   1,93 (s, 3H);
   1,78 (s, 3H);
   1,50 (d, 3H, J=5Hz);
   1,42 (d, 3H, J=6Hz)

### b) Verfahren zur Herstellung von 1.4-Dihydroxy-2.3.5-trimethyl-6-vinylbenzol

(2 g, 9,0 mmol) 2,4,5,7,8-Pentamethyl-4H-benzol[1,3]dioxin-6-ol wurden im Quarzrohr bei 460°C unter Vakuum (20 mmbar) pyrolysiert. Man erhielt einen hellbräunlichen Feststoff (1 g, 62%) mit einem Schmelzpunkt von 143°C-145°C.
¹H-NMR: (CDCI₃, 300 MHz) δ in ppm 6,67 (dd, 1H, J=12,5Hz, 19Hz);
   5,68 (d, 1 H, J=12,5Hz);
   5,50 (d, 1H, J=l9Hz);
   5,33 (s, 1 H);
   4,25 (s, 1 H);
   2,20 (s, 6H);
   2,13 (s, 3H)

### Beispiel 2

### a) Verfahren zur Herstellung von Essigsäure-2.4.5.7.8-pentamethyl-4H-benzol[1.3]dioxin-6-yl-ester

2,4,5,7,8-Pentamethyl-4H-benzol[1,3]dioxin-6-ol (117,4 g, 0,528 mol) und Triethylamin (63,14 g,0,624 mol) wurden in CH₂Cl₂ (800 ml) bei 0°C gelöst. Zu dieser Lösung wurde Acetylchlorid (49,0 g, 0,624 mol) während 1h zugetropft. Diese Mischung wurde 30 min. nachgerührt und dann mit Wasser (400ml versetzt. Die organische Phase wurde mit MgS0₄ getrocknet und unter Vakuum eingeengt. Ein leicht gelber Feststoff (130,1 g, 93%) mit einem Schmelzpunkt von 91,8-92,2°C wurde erhalten.
1 H-NMR: (CDCI₃, 300 MHz) δ in ppm 5,34 (q, 1 H, J=7,5Hz);
   4,98 (q, 1 H, J=7,5Hz);
   2,35 (s, 3H);
   2,13 (s, 3H);
   2,04 (s, 3H);
   1,95 (s, 3H);
   1,54 (d, 3H, J=7,5Hz);
   1,52 (d, 3H, J=7,5Hz)
Isomer: 5,20 (q, 1 H, J=7,5Hz);
   4,96 (q, 1 H, J=7,5Hz);
   2,35 (s, 3H);
   2.13 (s, 3H);
   2,04 (s, 3H);
   1,99 (s, 3H);
   1,54 (d, 3H, J=7,5Hz)
   1,47 (d, 3H, J=7,5Hz)

### b) Verfahren zur Herstellung von Essigsäure-4-hydroxy-2.5.6-trimethyl-3-vinylphenyl-ester

Essigsäure-2,4,5,7,8-pentamethyl-4H-benzol[1,3]dioxin-6-yl-ester (20 g, 75,6 mmol) wurden im Quarzrohr bei 450°C unter Vakuum (10 mbar) pyrolysiert. Nach Umkristallisation aus Hexan (65 ml) wurde ein weisser Feststoff (14,80 g, 77%) mit einem Schmelzpunkt von 73,5-74,8°C erhalten.
¹ H-NMR: (CDCI₃, 400 MHz) δ in ppm 6,63 (dd, 1 H, J=11,5 & 18,2Hz);
   5,69 (dd, 1H, J=1,8 & 11,5Hz);
   5,58 (s, 1 H);
   5,51 (dd, 1H, J=1,8 & 18,2Hz);
   2,32 (s, 3H);
   2,17 (s, 3H);
   2,05 (s, 3H);
   2,01 (s, 3H)

## Patentansprüche (Patentansprüche für folgende(n) Vertragsstaat(en) : AT, BE, CH, DE, DK, FR, GB, GR, IE, IT, LI, LU, NL, PT, SE)

1. Verfahren zur Herstellung von substituierten Vinylbenzolen der allgemeinen Formel 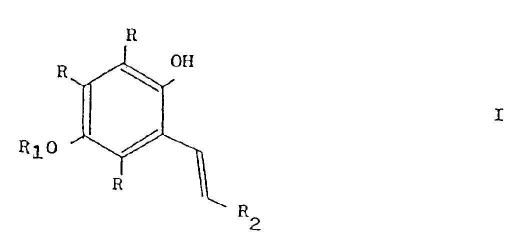 worin R eine Niederalkylgruppe mit 1 bis 4 C-Atomen, R₁ Wasserstoff oder eine Acetylgruppe und R₂ Wasserstoff, eine Niederalkylgruppe mit 1 bis 4 C-Atomen oder eine Benzylgruppe bedeutet, dadurch gekennzeichnet, dass ein Trialkylhydrochinon der allgemeinen Formel 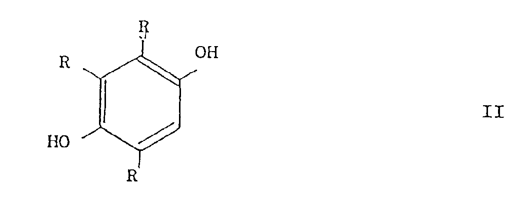 worin R die genannte Bedeutung hat, mit einem Aldehyd der allgemeinen Formel 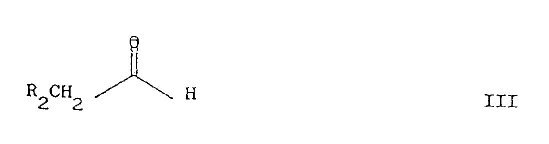 worin R₂ die genannte Bedeutung hat, in Gegenwart einer Säure unter Ausschluss von Wasser zu einem Acetal der allgemeinen Formel 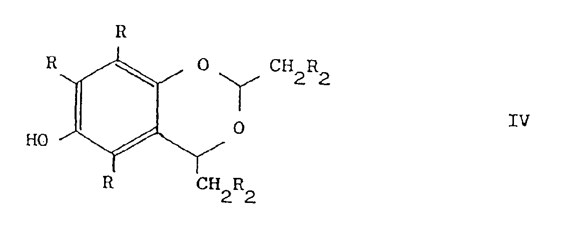 worin R und R₂ die genannte Bedeutung haben, umgesetzt wird, dieses Acetal gegebenenfalls mit Acetylchlorid zum acetylierten Acetal der allg. Formel 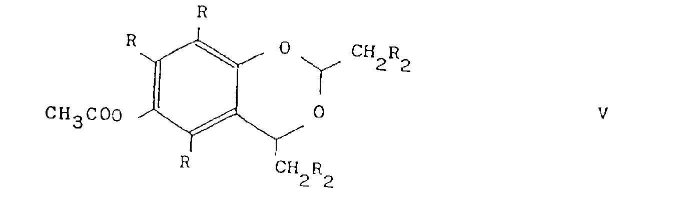 worin Rund R₂die genannte Bedeutung haben, umgesetzt wird und schliesslich die Acetale der allgemeinen Formel IV oder V zu einem Vinylbenzol der allgemeinen Formel 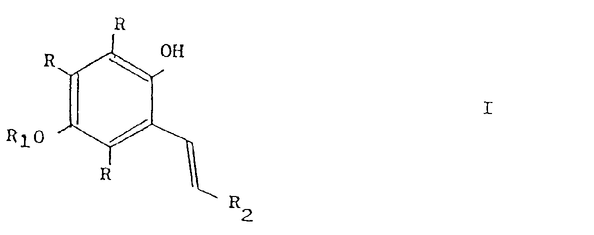 worin R, R₁ und R₂ die genannte Bedeutung haben, pyrolysiert werden.

2. Verfahren nach Patentanspruch 1, dadurch gekennzeichnet, dass als Trialkylhydrochinon der allgemeinen Formel II das Trimethylderivat mit R = CH₃ und als Aldehyd der allgemeinen Formel III der Acetaldehyd mit R₂ = Wasserstoff eingesetzt werden.

3. Verfahren nach Patentanspruch 1 oder 2, dadurch gekennzeichnet, dass die Umsetzung zum Acetal der allgemeinen Formel IV bei einer Temperatur zwischen -30°C und 30°C in Gegenwart eines inerten Lösungsmittels durchgeführt wird.

4. Verfahren nach einem der Patentansprüche 1 bis 3, dadurch gekennzeichnet, dass als Säure für die Umsetzung zum Acetal der allgemeinen Formel IV Chlorwasserstoff verwendet wird.

5. Verfahren nach einem der Patentansprüche 1 bis 4, dadurch gekennzeichnet, dass die Acetylierung zum Acetal der allgemeinen Formel V mit Acetylchlorid in Gegenwart eines tertiären Amins bei einer Temperatur zwischen 0°C und 100°C erfolgt.

6. Verfahren nach Patentanspruch 1, dadurch gekennzeichnet, dass die Pyrolyse der Acetale der allgemeinen Formel IV oder V bei einer Temperatur von über 300°C unter reduziertem Druck erfolgt.

7. Verfahren nach Patentanspruch 6, dadurch gekennzeichnet, dass die Pyrolyse des Acetals der allgemeinen Formel IV oder V bei einer Temperatur zwischen 400°C und 500°C und einem reduzierten Druck zwischen 0,5 und 100 mbar erfolgt.

8. Acetale der allgemeinen Formel 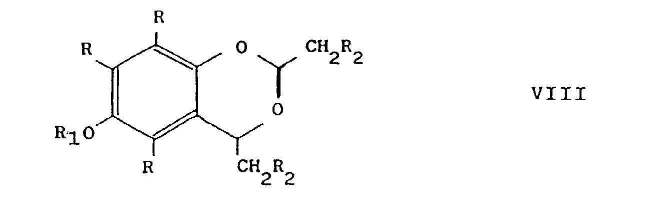 worin R, R₁ und R₂ die genannte Bedeutung haben.

9. 2,4,5,7,8-Pentamethyl-4H[1,3]dioxin-ol der Formel

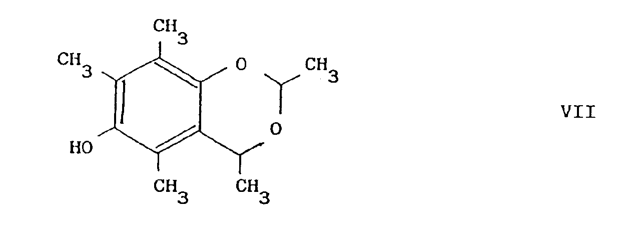

10. Substituierte Vinylbenzole der allgemeinen Formel 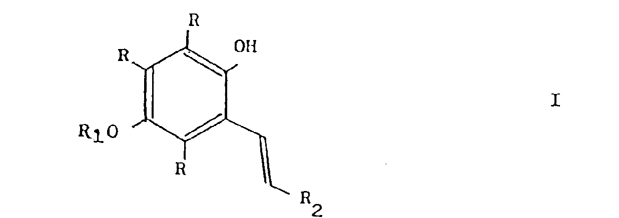 worin R, R₁ und R₂ die genannte Bedeutung haben, mit der Maßgabe, daß R₁ und R₂ nicht gleichzeitig Wasserstoff bedeuten.

11. Essigsäure-4-hydroxy-2,5,6-trimethyl-3-vinylphenylester

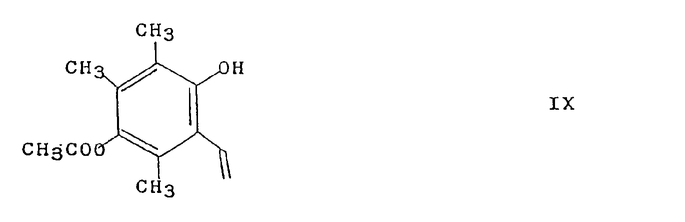

## Patentansprüche (Patentansprüche für folgende(n) Vertragsstaat(en) : ES)

1. Verfahren zur Herstellung von substituierten Vinylbenzolen der allgemeinen Formel 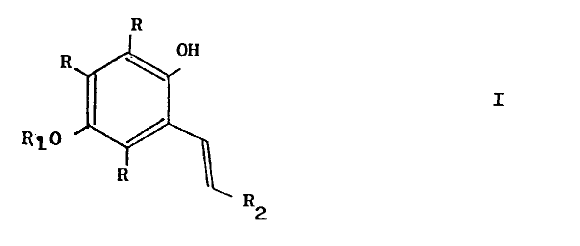 worin R eine Niederalkylgruppe mit 1 bis 4 C-Atomen, R₁ Wasserstoff oder eine Acetylgruppe und R₂ Wasserstoff, eine Niederalkylgruppe mit 1 bis 4 C-Atomen oder eine Benzylgruppe bedeutet, dadurch gekennzeichnet, dass ein Trialkylhydrochinon der allgemeinen Formel 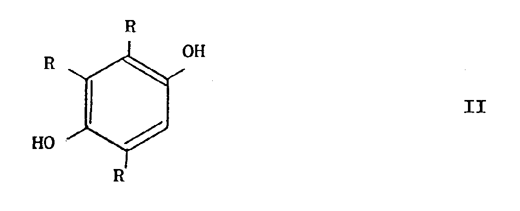 worin R die genannte Bedeutung hat, mit einem Aldehyd der allgemeinen Formel 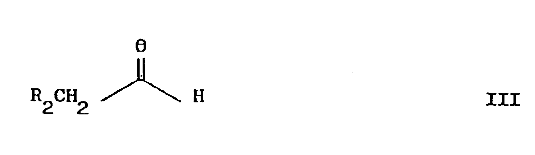 worin R₂ die genannte Bedeutung hat, in Gegenwart einer Säure unter Ausschluss von Wasser zu einem Acetal der allgemeinen Formel 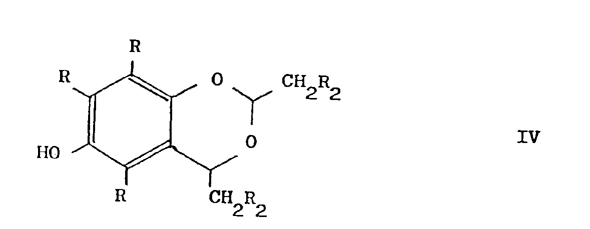 worin R und R₂ die genannte Bedeutung haben,umgesetzt wird, dieses Acetal gegebenenfalls mit Acetylchlorid zum acetylierten Acetal der allg. Formel 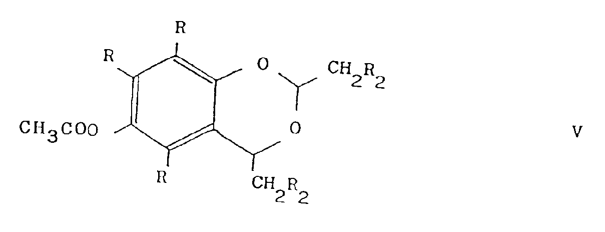 worin Rund R₂die genannte Bedeutung haben,umgesetzt wird und schliesslich die Acetale der allgemeinen Formel IV oder V zu einem Vinylbenzol der allgemeinen Formel 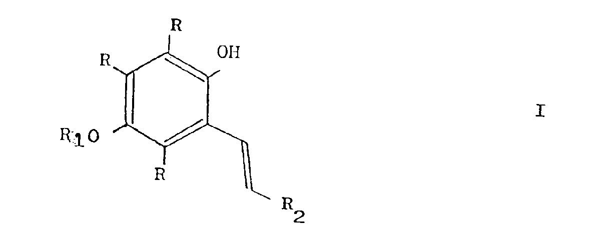 worin R, R₁ und R₂ die genannte Bedeutung haben, pyrolysiert werden.

2. Verfahren nach Patentanspruch 1, dadurch gekennzeichnet, dass als Trialkylhydrochinon der allgemeinen Formel II das Trimethylderivat mit R = CH₃ und als Aldehyd der allgemeinen Formel I der Acetaldehyd mit R₂ = Wasserstoff eingesetzt werden.

3. Verfahren nach Patentanspruch 1 oder 2, dadurch gekennzeichnet, dass die Umsetzung zum Acetal der allgemeinen Formel IV bei einer Temperatur zwischen -30°C und 30°C in Gegenwart eines inerten Lösungsmittels durchgeführt wird.

4. Verfahren nach einem der Patentansprüche 1 bis 3, dadurch gekennzeichnet, dass als Säure für die Umsetzung zum Acetal der allgemeinen Formel IV Chlorwasserstoff verwendet wird.

5. Verfahren nach einem der Patentansprüche 1 bis 4, dadurch gekennzeichnet, dass die Acetylierung zum Acetal der allgemeinen Formel V mit Acetylchlorid in Gegenwart eines tertiären Amins bei einer Temperatur zwischen 0°C und 100°C erfolgt.

6. Verfahren nach Patentanspruch 1, dadurch gekennzeichnet, dass die Pyrolyse der Acetale der allgemeinen Formel IV oder V bei einer Temperatur von über 300°C unter reduziertem Druck erfolgt.

7. Verfahren nach Patentanspruch 6, dadurch gekennzeichnet, dass die Pyrolyse des Acetals der allgemeinen Formel IV oder V bei einer Temperatur zwischen 400°C und 500°C und einem reduzierten Druck zwischen 0,5 und 100 mbar erfolgt.

8. Verfahren nach einem der Patentansprüche 1 bis 7, dadurch gekennzeichnet, dass es sich bei dem Acetal der allgemeinen Formel IV um 2,4,5,7,8-Pentamethyl-4H[1,3]dioxin-ol der Formel 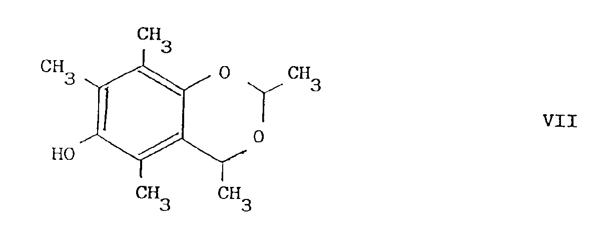 handelt.

9. Verfahren nach einem der Patentansprüche 1 bis 7, dadurch gekennzeichnet, dass im substituierten Vinylbenzol der allgemeinen Formel 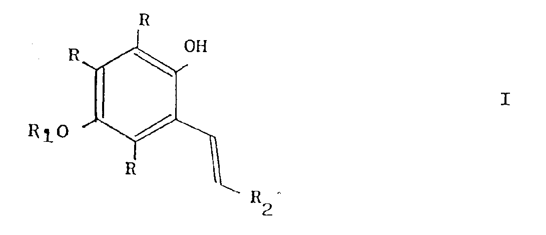 worin R, R₁ und R₂ die genannte Bedeutung haben, R₁ und R₂ nicht gleichzeitig Wasserstoff bedeuten.

10. Verfahren nach Patentanspruch 9, dadurch gekennzeichnet, dass es sich bei dem substituierten Vinylbenzol der allgemeinen Formel 1 um Essigsäure-4-hydroxy-2,5,6-trimethyl-3-vinylphenylester der Formel 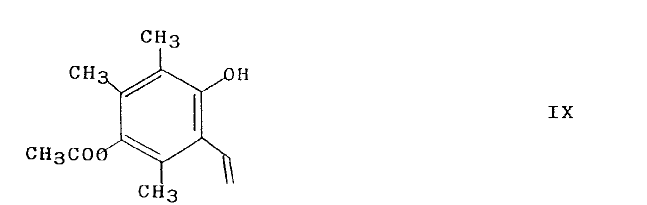 handelt.

## Claims (Claims for the following Contracting State(s) : AT, BE, CH, DE, DK, FR, GB, GR, IE, IT, LI, LU, NL, PT, SE)

1. A process for the preparation of substituted vinylbenzenes of general formula: 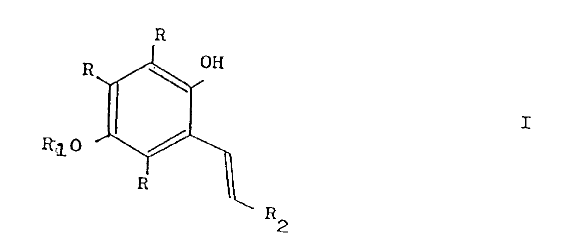 wherein R is a lower alkyl group having 1 to 4 C atoms, R₁ is hydrogen or an acetyl group and R₂ is hydrogen, a lower alkyl group having 1 to 4 C atoms or a benzyl group, characterized in that a trialkylhydroquinone of the general formula: 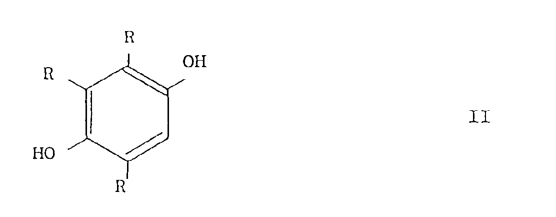 wherein R has the above-mentioned meaning, is reacted with an aldehyde of the general formula: 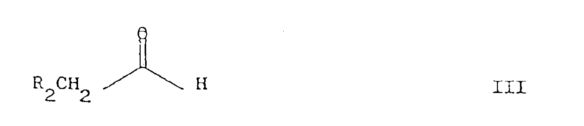 wherein R₂ has the above-mentioned meaning, in the presence of an acid and with exclusion of water to the acetal of the general formula: 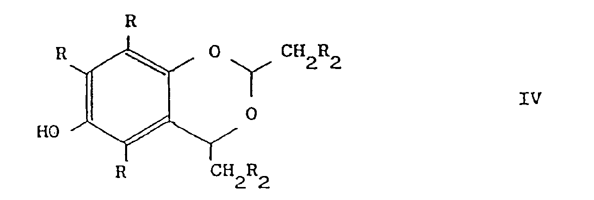 wherein R and R₂ have the above-mentioned meanings, and this acetal, if desired, is reacted with acetyl chloride to the acetylated acetal of the general formula: 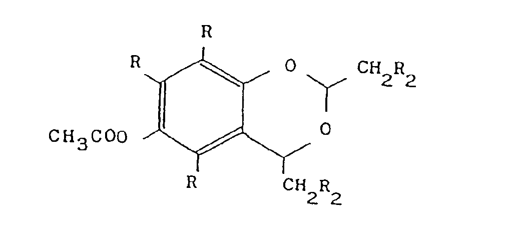 wherein R and R₂ have the above-mentioned meanings, and finally the acetals of the general formula IV or V are pyrolyzed to a vinylbenzene of the general formula: 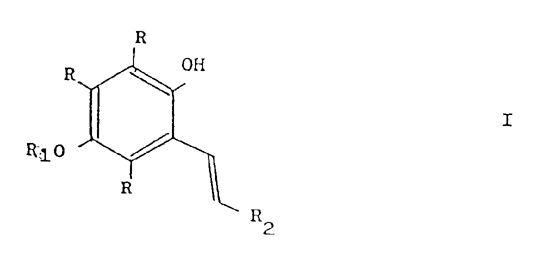 wherein R, R₁ and R₂ have the above-mentioned meanings.

2. The process of claim 1, characterized in that the trimethyl derivative with R = CH₃ is employed as the trialkylhydroquinone of the general formula II and acetaldehyde with R₂ = hydrogen is employed as the aldehyde of the general formula III.

3. The process of claim 1 or 2, characterized in that the reaction to the acetal of the general formula IV is conducted at a temperature of from -30°C to 30°C in the presence of an inert solvent.

4. The process of one of the claims 1 to 3, characterized in that hydrogen chloride is employed as the acid used for the reaction to the acetal of the general formula IV.

5. The process of one of the claims 1 to 4, characterized in that the acetylation to the acetal of the general formula V takes place with acetyl chloride in the presence of a tertiary amine at a temperature of from 0°C to 100°C.

6. The process of claim 1, characterized in that the pyrolysis of the acetals of the general formula IV or V takes place at a temperature above 300°C, under reduced pressure.

7. The process of claim 6, characterized in that the pyrolysis of the acetal of the general formula IV or V takes place at a temperature of from 400°C to 500°C and a reduced pressure of from 0.5 to 100 mbar.

8. Acetals of general formula 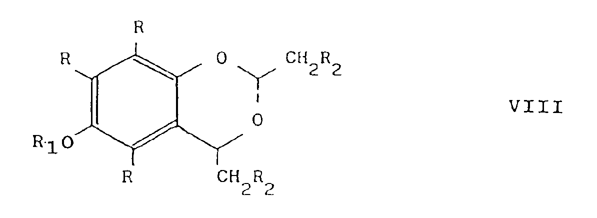 wherein R, R₁ and R₂ have the above-mentioned meanings.

9. 2,4,5,7,8-Pentamethyl-4H[1,3]dioxin-ol of formula

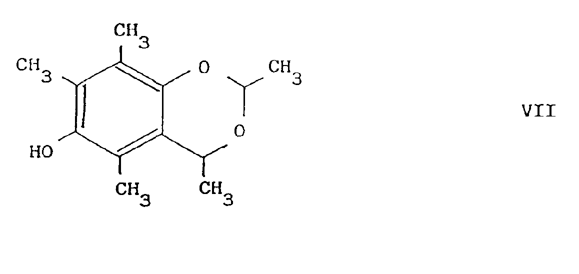

10. Substituted vinylbenzenes of general formula 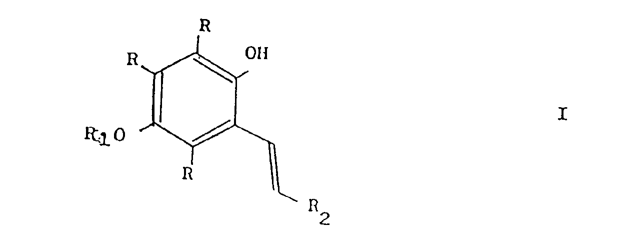 wherein R, R₁ and R₂ have the above-mentioned meanings, with the proviso that R₁ and R₂ may not simultaneously represent hydrogen.

11. Acetic acid 4-hydroxy-2,5,6-trimethyl-3-vinylphenyl ester

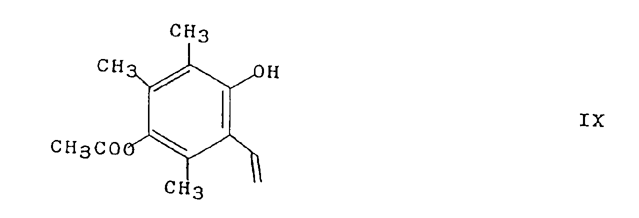

## Claims (Claims for the following Contracting State(s) : ES)

1. A process for the preparation of substituted vinylbenzenes of general formula: 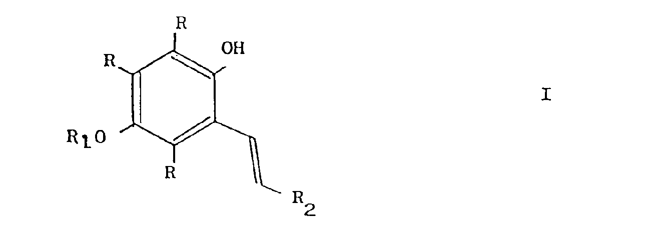 wherein R is a lower alkyl group having 1 to 4 C atoms, R₁ is hydrogen or an acetyl group and R₂ is hydrogen, a lower alkyl group having 1 to 4 C atoms or a benzyl group, characterized in that a trialkylhydroquinone of the general formula: 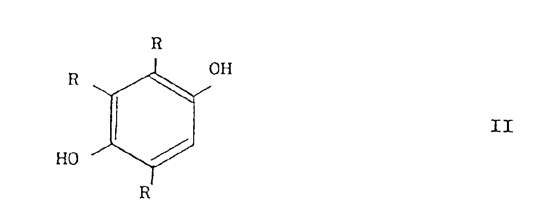 wherein R has the above-mentioned meaning, is reacted with an aldehyde of the general formula: 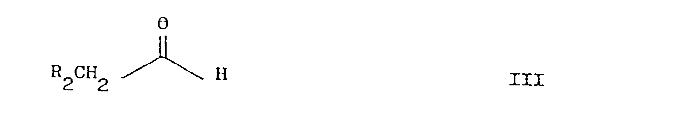 wherein R₂ has the above-mentioned meaning, in the presence of an acid and with exclusion of water to the acetal of the general formula: 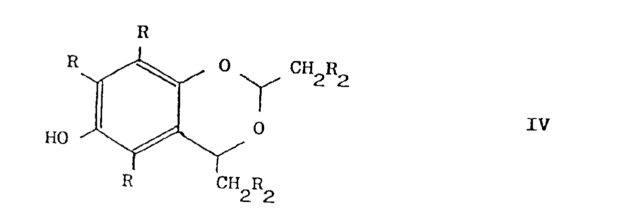 wherein R and R₂ have the above-mentioned meanings, and this acetal, if desired, is reacted with acetyl chloride to the acetylated acetal of the general formula: 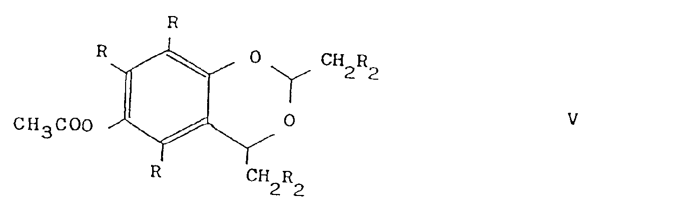 wherein R and R₂ have the above-mentioned meanings, and finally the acetals of the general formula IV or V are pyrolyzed to a vinylbenzene of the general formula: 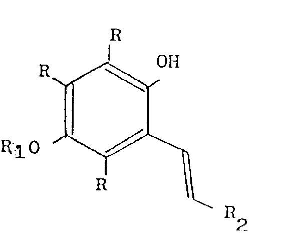 wherein R, R₁ and R₂ have the above-mentioned meanings.

2. The process of claim 1, characterized in that the trimethyl derivative with R = CH₃ is employed as the trialkylhydroquinone of the general formula II and acetaldehyde with R₂ = hydrogen is employed as the aldehyde of the general formula III.

3. The process of claim 1 or 2, characterized in that the reaction to the acetal of the general formula IV is conducted at a temperature of from -30°C to 30°C in the presence of an inert solvent.

4. The process of one of the claims 1 to 3, characterized in that hydrogen chloride is employed as the acid used for the reaction to the acetal of the general formula IV.

5. The process of one of the claims 1 to 4, characterized in that the acetylation to the acetal of the general formula V takes place with acetyl chloride in the presence of a tertiary amine at a temperature of from 0°C to 100°C.

6. The process of claim 1, characterized in that the pyrolysis of the acetals of the general formula IV or V takes place at a temperature above 300°C, under reduced pressure.

7. The process of claim 6, characterized in that the pyrolysis of the acetal of the general formula IV or V takes place at a temperature of from 400°C to 500°C and a reduced pressure of from 0.5 to 100 mbar.

8. The process of one of the claims 1 to 7, characterized in that the acetal of the general formula IV is 2,4,5,7,8-pentamethyl-4H[1,3]dioxine-ol of formula

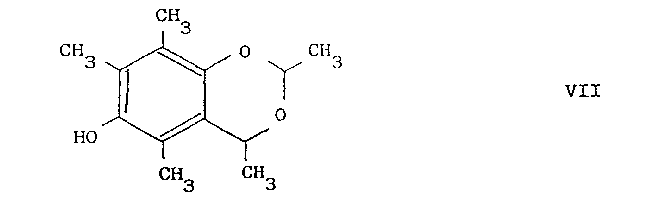

9. The process of one of the claims 1 to 7, characterized in that, in the substituted vinyl benzene of general formula 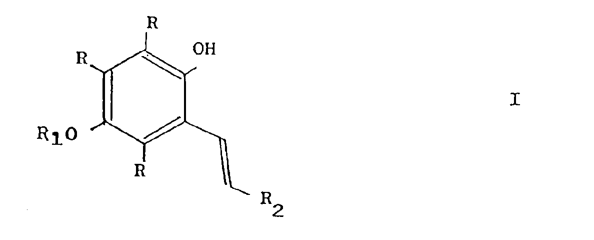 wherein R, R₁ and R₂ have the above-mentioned meanings, R₁ and R₂ may not simultaneously represent hydrogen.

10. The process of claim 9, characterized in that the substituted vinylbenzene of general formula I is acetic acid 4-hydroxy-2,5,6-trimethyl-3-vinylphenyl ester of formula

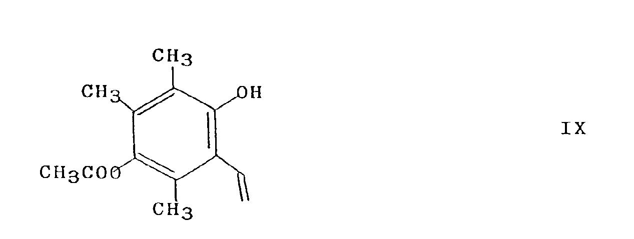

## Revendications (Revendications pour l'(les) Etat(s) contractant(s) suivant(s) : AT, BE, CH, DE, DK, FR, GB, GR, IE, IT, LI, LU, NL, PT, SE)

1. Procédé pour la préparation de benzols de vinyle ou styrènes substitués de la formule générale 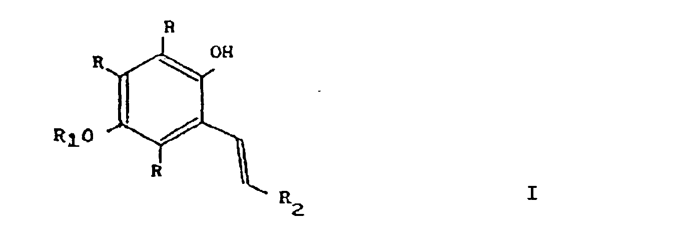 dans laquelle R signifie un groupe alkyle inférieur avec 1 à 4 atomes C, R₁ l'hydrogène ou un groupe acétyle et R₂ l'hydrogène, un groupe alkyle inférieur avec 1 à 4 atomes C ou un groupe benzyle, caractérisé en ce que l'on transforme une trialkylhydroquinone de la formule générale 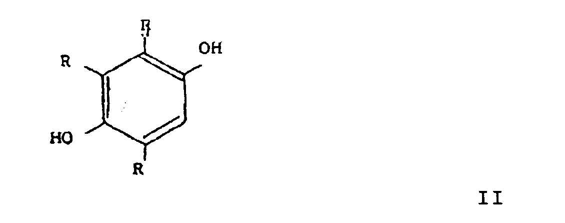 dans laquelle R a la signification indiquée, avec un aldéhyde de la formule générale 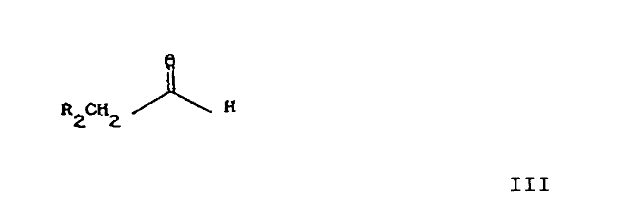 dans laquelle R₂ a la signification indiquée, en présence d'un acide sans eau, en un acétal de la formule générale 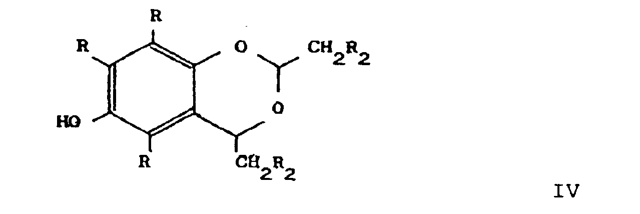 dans laquelle R et R₂ ont la signification indiquée, cet acétal est éventuellement transformé avec le chlorure d'acétyle en l'acétal acétylé de la formule générale 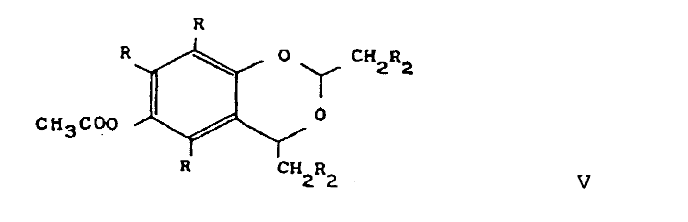 dans laquelle R et R₂ ont la signification indiquée, et enfin on pyrolyse les acétals de la formule générale IV ou V en un benzol de vinyle ou styrène de la formule générale 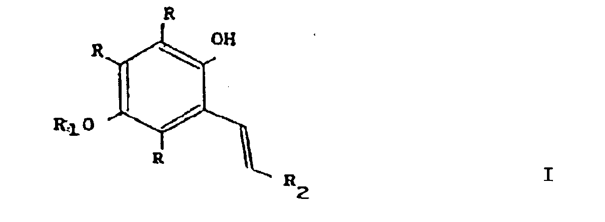 dans laquelle R, R₁ et R₂ ont la signification indiquée.

2. Procédé selon la revendication 1, caractérisé en ce qu'en tant que trialkylhydroquinone de la formule générale 11, on met en oeuvre le dérivé de triméthyle avec R = CH₃, et comme aldéhyde de la formule générale III l'acétaldéhyde avec R₂ = hydrogène.

3. Procédé selon la revendication 1 ou 2, caractérisé en ce que la transformation en acétal de la formule générale IV est effectuée à une température entre -30°C et 30°C, en présence d'un solvant inerte.

4. Procédé selon l'une des revendications 1 à 3, caractérisé en ce qu'en tant qu'acide pour la transformation en acétal de la formule générale IV, on utilise du chlorure d'hydrogène.

5. Procédé selon l'une des revendications 1 à 4, caractérisé en ce que l'acétylation en acétal de la formule générale V s'effectue avec du chlorure d'acétyle en présence d'une amine tertiaire, à une température entre 0°C et 100°C.

6. Procédé selon la revendication 1, caractérisé en ce que la pyrolyse des acétals de la formule générale IV ou V, s'effectue à une température supérieure à 300°C sous pression réduite.

7. Procédé selon la revendication 6, caractérisé en ce que la pyrolyse des acétals de la formule générale IV ou V, s'effectue à une température entre 400°C et 500°C et à une pression réduite entre 0,5 et 100 mbars.

8. Acétals de la formule générale 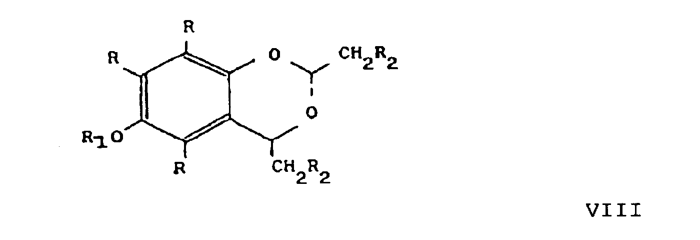 dans laquelle R, R₁ et R₂ ont la signification indiquée.

9. 2, 4, 5, 7, 8-pentaméthyl-4H[1,3]dioxin-ol de la formule

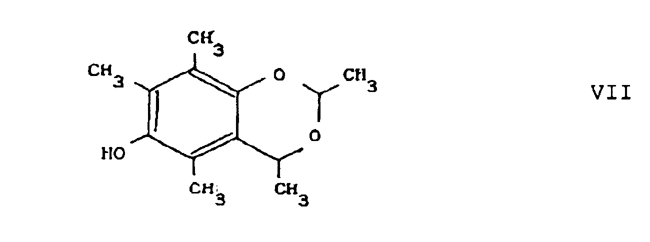

10. Benzol de vinyle substitué de la formule générale 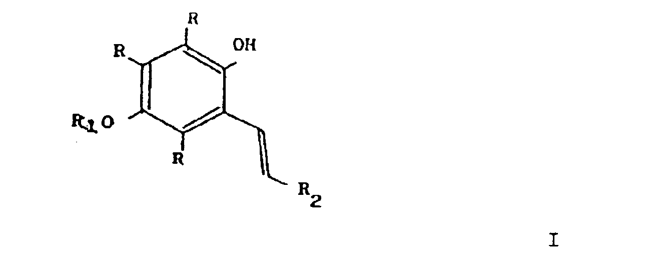 dans laquelle R, R₁ et R₂ ont la signification indiquée, dans la mesure où R₁ et R₂ ne signifient pas simultanément l'hydrogène.

11. Acide acétique-4-hydroxy-2,5,6-triméthyl-3-vinylphénylester

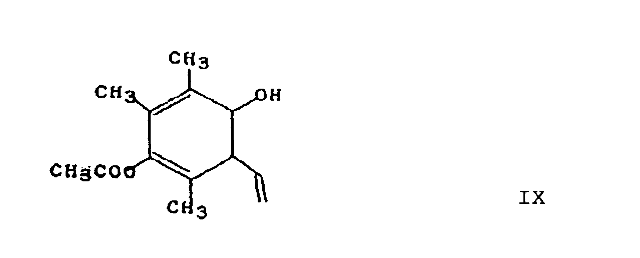

## Revendications (Revendications pour l'(les) Etat(s) contractant(s) suivant(s) : ES)

1. Procédé pour la préparation de benzols de vinyle ou styrènes substitués de la formule générale 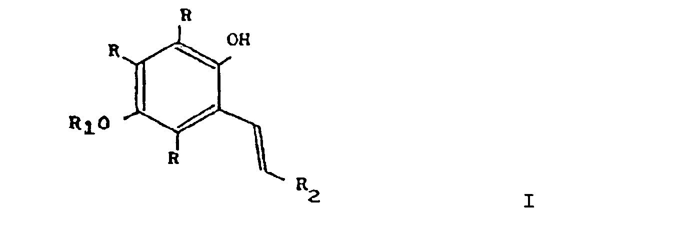 dans laquelle R signifie un groupe alkyle inférieur avec 1 à 4 atomes C, R₁ l'hydrogène ou un groupe acétyle et R₂ l'hydrogène, un groupe alkyle inférieur avec 1 à 4 atomes C ou un groupe benzyle, caractérisé à ce que l'on transforme une trialkylhydroquinone de la formule générale 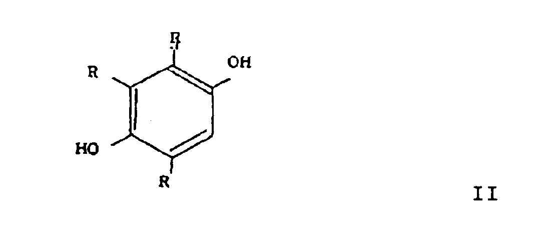 dans laquelle R a la signification indiquée, avec un aldéhyde de la formule générale 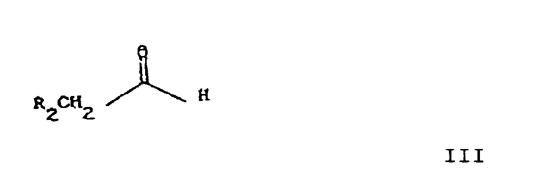 dans laquelle R₂ a la signification indiquée, en présence d'un acide sans eau, en un acétal de la formule générale 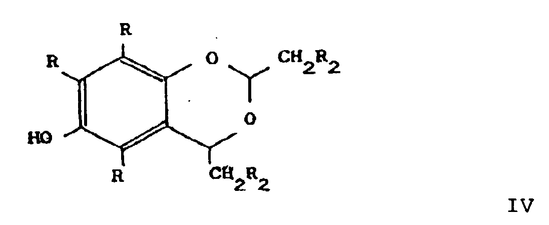 dans laquelle R et R₂ ont la signification indiquée, cet acétal est éventuellement transformé avec le chlorure d'acétyle en l'acétal acétylé de la formule générale 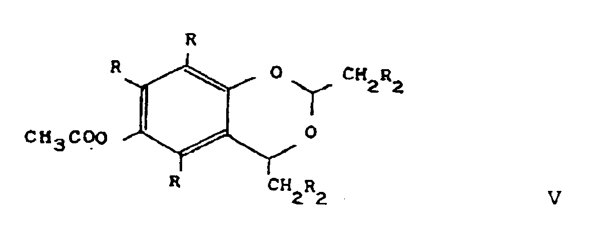 dans laquelle R et R₂ ont la signification indiquée, et enfin on pyrolyse les acétals de la formule générale IV ou V en un benzol de vinyle ou styrène de la formule générale 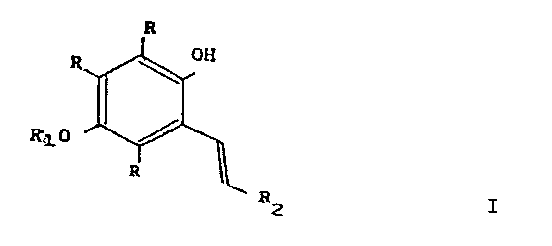 dans laquelle R, R₁ et R₂ ont la signification indiquée.

2. Procédé selon la revendication 1, caractérisé en ce qu'en tant que trialkylhydroquinone de la formule générale II, on met en oeuvre le dérivé de triméthyle avec R = CH₃, et comme aldéhyde de la formule générale III l'acétaldéhyde avec R₂ = hydrogène.

3. Procédé selon la revendication 1 ou 2, caractérisé en ce que la transformation en acétal de la formule générale IV est effectuée à une température entre -30°C et 30°C, en présence d'un solvant inerte.

4. Procédé selon l'une des revendications 1 à 3, caractérisé en ce qu'en tant qu'acide pour la transformation en acétal de la formule générale IV, on utilise du chlorure d'hydrogène.

5. Procédé selon l'une des revendications 1 à 4, caractérisé en ce que l'acétylation en acétal de la formule générale V s'effectue avec du chlorure d'acétyle en présence d'une amine tertiaire, à une température entre 0°C et 100°C.

6. Procédé selon la revendication 1, caractérisé en ce que la pyrolyse des acétals de la formule générale IV ou V, s'effectue à une température supérieure à 300°C sous pression réduite.

7. Procédé selon la revendication 6, caractérisé en ce que la pyrolyse des acétals de la formule générale IV ou V s'effectue à une température entre 400°C et 500°C, et à une pression réduite entre 0,5 et 100 mbars.

8. Procédé selon l'une des revendications 1 à 7, caractérisé en ce qu'il s'agit pour l'acétal de la formule générale IV de 2,4,5,7,8-pentaméthyl-4H[1,3]dioxin-ol de la formule générale

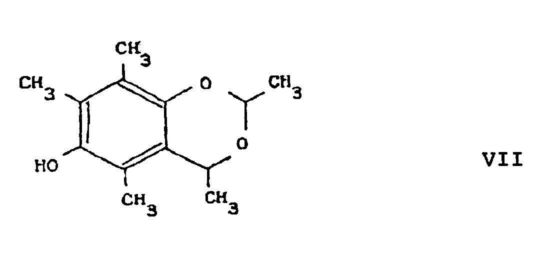

9. Procédé selon l'une des revendications 1 à 7, caractérisé en ce que dans le benzol de vinyle substitué de la formule générale dans laquelle R, R₁ et R₂ ont la signification indiquée, R₁ et R₂ ne signifient pas simultanément l'hydrogène.

10. Procédé selon la revendication 9, caractérisé en ce qu'il s'agit, pour le benzol de vinyle substitué de la formule générale l, d'acide acétique-4-hydroxy-2,5,6-triméthyl-3-vinylphénylester de la formule
